# EUROPEAN PATENT APPLICATION

(11) **EP 0 796 659 A2**
(43) Date of publication of application: **24.09.1997**
(21) Application number: 97301415.2
(22) Date of filing: 04.03.1997
(51) Int. Cl.: B01L 3/02, A61B 5/14

(54) **Pipette for collecting and dispensing material samples**

(30) Priority: 22.03.1996 US 620451
(71) Applicant: SERIM RESEARCH CORPORATION, Elkhart, Indiana 46514-0002 (US)
(72) Inventor: Crawford, Ferdon H., Elkhart, Indiana 46514 (US)
(74) Representative: Perry, Robert Edward

(57) **Abstract**

The present invention relates to a unitary volumetric pipette (1) for collecting and dispensing a desired measured sample volume. The pipette (1) comprises a stem portion (6), narrow diameter bulb portion (9), and a wide diameter bulb portion (11). The inner chamber of the stem portion (6) has a volume corresponding to the desired sample volume and the inner diameter and length of the stem portion (6) is dimensioned according to the desired chamber volume. The operator can collect a precise known sample volume equal to the inner volume of the stem (6). The narrow bulb portion (9) gives the operator greater accuracy in drawing the sample and the wide bulb portion (11) permits the operator to dispense the entire sample from the pipette (1). The pipette (1) design of the present invention can be manufactured quickly and inexpensively using conventional blowmolding methods. Using the pipette design of the present invention, an operator can easily and efficiently collect, transport and dispense a precise, small sample volume. This pipette design is particularly suited for taking capillary blood samples from a finger or heel stick for a single diagnostic test.

## Description

The present invention relates to pipettes for collecting, transporting and dispensing liquid samples.

Pipettes are well known devices designed to collect, transport and dispense uniform, known amounts of a liquid sample. As a tool for handling a determinable volume of a liquid sample, pipettes are widely used in the medical field, especially for handling liquid samples in connection with laboratory testing.

With regard to laboratory testing, pipettes are particularly useful for collecting, transporting and dispensing a volume of blood sample from a sample source to a test location. The blood sample volume required for laboratory testing varies, and for a large number of diagnostic tests, only a small sample of blood is necessary. For such tests, the amount of blood drawn from the patient should be kept to a minimum in order reduce the patient's pain and discomfort.

A small blood sample required for a single diagnostic test is usually drawn from the patient by using a finger or heel stick rather than by using a device comprising a needle attached to a collecting tube. A finger or heel stick creates a blood sample by puncturing the surface of the skin only to the depth necessary to draw capillary blood to the surface of the skin. The depth of the puncture must be kept to a minimum to reduce the patient's pain and discomfort. This is especially important when the patient is a small child or an infant, not only to reduce the child's discomfort, but also to minimize the movement of the finger or heel while the sample is being collected.

Using a finger or heel stick, a sample of blood can be collected directly from a patient, transported to and dispensed at a test location using a single pipette. However, since the available sample volume is small, and the patient may be experiencing discomfort during the sample collection, the pipette must be designed to allow quick and accurate collection of a small sample volume.

The volume of sample drawn into and dispensed from a pipette is generally determined by monitoring the level of the sample against calibration marks on the stem or storage portion of the pipette. A sample is generally drawn using air displacement mechanisms. The operator squeezes a flexible bulb portion of the pipette, usually an attached rubber bulb, to expel a volume of air inside the pipette, places the open tip of the stem portion against the sample, and slowly releases the pressure to create an air pressure differential between the interior and exterior of the pipette to draw the sample into the pipette. A mechanical attachment may also be used in place of a flexible bulb to displace a volume of air inside the pipette.

To ensure that the proper sample volume is collected and dispensed, the operator must carefully manipulate the bulb while monitoring the level of the sample against calibration marks disposed on the outside wall of the pipette. However, it is impractical to use conventional pipettes that are designed to manipulate moderate sample volumes when collecting a small sample volume from a finger or heel stick. It becomes awkward and impractical to monitor the level of a small sample volume in a pipette since the level may be difficult to see and the calibration marks may not allow proper discrimination between small sample volumes. Additionally, it may be impossible operate the flexible bulb with sufficient delicacy to precisely manipulate a small sample volume within a relatively large stem. The need to quickly and accurately collect a small sample volume is particularly problematic when collecting a capillary blood sample directly from a patient because only a limited sample quantity is available, drawing time must be kept to a minimum, and the patient may not keep the finger or heel motionless while the sample is being collected.

A number of different pipette designs are used facilitate collecting and dispensing a small sample volume. One pipette design uses the capillary effect rather than air displacement to draw a predetermined sample volume into the pipette. Capillary effect pipettes are generally constructed using glass stems and require that the inner diameter of the stems be small enough to ensure that the capillary affect will draw the sample into the stem.

The capillary effect pipette draws the sample into the stem by using the forces created by the surface tension of the sample. The inner diameter of the stem determines the height of the sample drawn into the stem, and thereby the volume of sample drawn. The sample is expelled from the pipette using an air displacement attachment. The problems associated with capillary effect pipettes include the increased cost and complexity of manufacturing the glass stems having sufficiently small diameters and the risk of glass stem breakage. Also, additional attachments are required to draw the sample further into the pipette in order to allow pipette to be used as a transport means to safely transfer the sample to a test location, and to allow the operator to expel the sample from the pipette.

Another pipette design uses a stem portion that partially extends into a larger diameter bulb portion to create an overflow space in the bulb portion. In such a pipette, any liquid sample collected in excess of the inner volume of the stem flows into the overflow space and a known sample volume, corresponding to the inner volume of the stem portion, remains in the stem portion. When the operator dispenses the sample, only that sample volume contained in the stem portion is dispensed.

The overflow pipette design is not particularly useful for efficiently collecting small sample volumes. This design requires a sufficiently large sample volumes to be available to create a sample overflow in the large diameter bulb portion. If the overflow condition is not created, the operator must rely on the calibration marks on the stem to measure the sample volume collected and dispensed. This pipette also complicates the manufacturing process because the stem portion must extend some predetermined length into the bulb portion.

Yet another pipette design that uses the inner volume of the stem to measure the sample volume requires the pipette to be made by bonding components that have been separately made. By using separate manufacturing methods for different components of the pipette, the components that require precise dimensions may be formed using more accurate molding processes whereas components that do not require precise dimensions may be formed using less accurate molding processes. The separate components are bonded together to form a single device having a sufficiently strong seal to prevent fluid leakage. The problem with this pipette design is the additional cost and manufacturing complexity required due to separately forming each component using different processes, bonding the separate components together and ensuring a leak proof seal.

In view of the above, there is a need for an improved pipette to collect and dispense a precise, known sample volume. The pipette must be easy to use and capable of manipulating a small sample volume with sufficient accuracy. The pipette must also be capable of being manufactured simply, quickly and inexpensively.

In particular, there is a need for an improved pipette to collect, transport and dispense a small blood sample, wherein the small blood sample is a capillary blood sample taken from a finger or heel stick, and the sample volume is sufficient to perform a single diagnostic test. The pipette must allow the operator to easily and quickly collect a small precise sample from a patient in order to minimize patient discomfort. The pipette must also serve as a means for safely transporting the sample from the collection point to the test location and allow the operator to fully dispense the sample contained within.

The present invention provides a simple and inexpensive unitary pipette for collecting, transporting, and dispensing a small, precise, predetermined sample volume. The inventional pipette comprises a generally cylindrical stem portion having an inner chamber corresponding to the desired sample volume integrally connected to a narrow diameter bulb portion and a larger diameter bulb portion. The narrow diameter bulb portion provides additional structural stiffness as well as finer control while drawing the sample and the larger diameter portion permits the operator to fully dispense the sample contained in the pipette.

The pipette of the present invention is particularly suited for accurately collecting, transporting, and dispensing blood samples from a finger or heel stick and used for diagnostic tests. The simple construction of the pipette of the present invention permits the pipette to be manufactured quickly and inexpensively. Thus, the pipette of the present invention eliminates many of the disadvantages associated with the cost and complexity of manufacturing the pipette arrangements described above.

The unitary pipette of the present invention comprises a stem having a length and diameter determined according to the desired sample volume operatively connected to a narrow diameter bulb portion and a wide diameter bulb portion. The pipette is constructed using materials which can be molded using conventional blow molding technology at low cost.

The inner volume of the stem, which is defined by the length and inner diameter of the stem, corresponds to the desired sample volume. The operator can collect an accurate sample volume by drawing a sufficient amount of sample to fill the inner volume of the stem. The operator knows that a particular sample volume necessary for a diagnostic test has been collected when the sample fills the entire inner volume of the stem, which is indicated by the alignment of the sample level with the upper end of the stem portion.

The narrow diameter bulb portion of the pipette of the present invention adds stiffness to the entire apparatus and gives the operator finer control when collecting or dispensing a sample. The narrow diameter portion gives the operator finer control since displacing a relatively smaller volume of air than the wide bulb portion allows the operator to more easily manipulate smaller liquid volumes.

When collecting the sample, the operator places the tip of the pipette near the sample to be collected and squeezes the narrow diameter portion to expel a volume of air from the pipette. The operator then places the open end of the stem against the sample and slowly releases the pressure to draw the sample into the stem. The operator monitors the sample volume by monitoring the sample level in the stem portion.

When the sample completely fills the stem portion, which is indicated by the alignment of the sample level with the upper end of the stem portion, the operator removes the tip of the pipette from the sample and further releases the pressure on the narrow diameter bulb portion of the pipette to draw the sample further into the pipette. As a result, one end of the sample is drawn into the narrow diameter bulb portion of the pipette and the opposite end of the sample is drawn away from the open end of the stem. Now the pipette can be used to safely transport the sample from the collection point to the test location. The sample is dispensed from the pipette by squeezing the wide portion of the bulb. The volume of air displaced by squeezing the wide diameter portion is sufficient to expel the entire sample held in the pipette.

Therefore, it is an objective of this invention to provide an inexpensive unitary volumetric pipette which can be used to quickly and easily collect, transport and dispense a known sample volume.

It is a further objective of this invention to provide an above described unitary volumetric pipette which can be easily manufactured using materials that can be molded using conventional blow molding methods.

It is a further specific objective of this invention to provide a unitary volumetric pipette to be used in connection with small capillary blood samples from a finger or heel stick.

The above-mentioned and other features and advantages of this invention, and the manner of attaining them, will become more apparent and the invention will be better understood by reference to the following description of an embodiment of the invention taken in conjunction with the accompanying drawings, wherein:
Fig. 1 is a perspective view of a unitary volumetric pipette made in accordance with the present invention;
Fig. 2 is a cross sectional view of the unitary volumetric pipette of Fig. 1;
Fig. 3 is a cross sectional view of the unitary volumetric pipette of Fig. 2 taken along line 3-3;
Figs. 4A-4G illustrate the sequence for collecting, transporting and dispensing a sample using the unitary volumetric pipette of the present invention.

Corresponding reference characters indicate corresponding parts throughout the several views. Although the drawings represent an embodiment of the present invention, the drawings are not necessarily to scale and certain features may be exaggerated in order to better illustrate and explain the present invention. The exemplification set out herein illustrates an embodiment of the invention, in one form, and such exemplification is not to be construed as limiting the scope of the invention in any manner.

The embodiment disclosed below is not intended to be exhaustive or limit the invention to the precise form disclosed in the following detailed description. Rather, the embodiment is chosen and described so that others skilled in the art may utilize its teaching.

This invention relates to a unitary volumetric pipette comprising a flexible material which can be molded using conventional molding methods. The range of possible materials include low density polyethylene, high density polyethylene and polypropylene. Low density polyethylene is used in the preferred embodiment. The pipette permits the operator to draw a small, accurate, known sample, use the pipette to transport the sample, and dispense the sample. The pipette comprises a stem portion, which is used to measure the sample volume, a narrow diameter bulb portion, which is used to draw the sample and a wide diameter bulb portion, which is used to dispense the sample.

The sample volume is determined by the inner diameter and the length of the stem. This invention is particularly suited for small sample volumes in the range of 2 to 30 microliters. This volume range is particularly useful for collecting and dispensing body fluids and reagents for diagnostic tests. The operator can easily monitor and verify the volume of sample collected by checking the level of the sample in the stem. The operator is assured that a sufficient sample volume is collected when the sample fills the inner volume of the stem, which is indicated by the alignment of the sample level with the upper end of the stem portion.

The inventional pipette is particularly useful for collecting, transporting, and dispensing small capillary blood samples from a finger or heel stick. Using the inventional pipette allows the blood sample to be quickly and easily collected from a finger stick or a heel stick without requiring a painful deep lance. The stem of the pipette can be manufactured according to the desired volume, such that the collected sample is sufficient to perform a single diagnostic test.

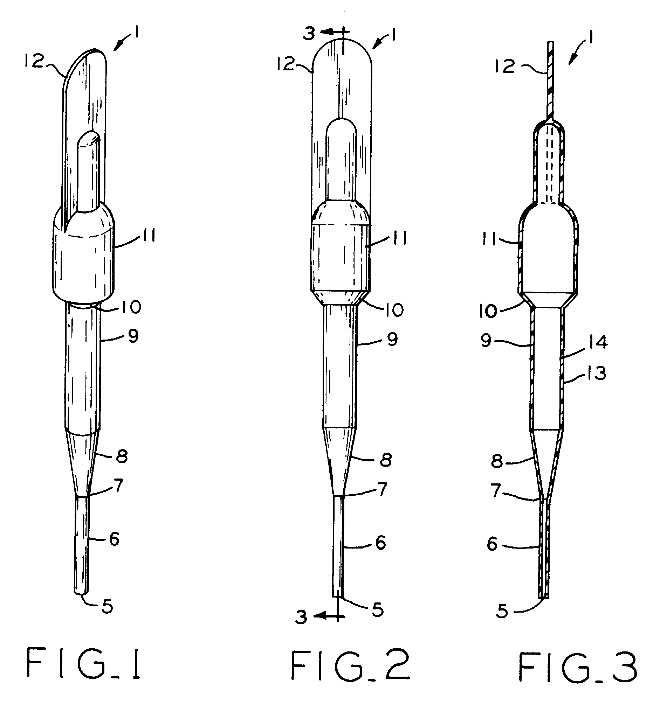

Referring to Figs. 1 and 2, volumetric pipette 1 comprises stem 6, small diameter bulb portion 9 and large diameter bulb portion 11. Unitary volumetric pipette 1 includes outer wall 13 and inner wall 14. Open tip 5 is disposed on one end of stem 6, and is designed to draw in a sample, while the other end of stem 6 is connected to small diameter portion 9. The sample drawn is bounded by the inner wall and ends of stem 6 and its volume is defined by the length and inner diameter of stem 6. The dimensions of stem 6 can be manufactured as necessary for different pipette sizes and sample volumes.

Small diameter bulb portion 9 is connected to stem 6 at one end and large diameter bulb portion 11 at the other end. Large diameter bulb portion 11 is connected to narrow diameter bulb portion 9 at one end and closed at the other end. Tab 12 is also disposed on one end of large diameter bulb portion 11 and provides a gripping area for the operator when transporting the sample from the collection point to the test location. Note that the two bulb portions have been labeled small diameter bulb portion and large diameter bulb portion to distinguish the relative diameters of the two bulb portion and is not intended to imply a particular diameter.

The inner volume of small diameter bulb portion 9 is sized to permit the operator to displace a sufficient volume of air to draw a sufficient sample volume to completely fill stem 6. The inner volume of large diameter bulb portion 11 is sized to permit the operator to fully dispense the sample from pipette 1. Small diameter bulb portion 9 gives the operator better control while drawing the sample because a relatively smaller volume of air is displaced compared to the volume of air displaced by the large diameter bulb portion. However, the inner volume of small diameter bulb portion 9 is still large enough to ensure that the sample is fully drawn into stem 6. Small diameter portion 9 also adds stiffness to the structure, which allows the device to be made from a soft plastic such as low density polyethylene and still provide structural robustness required for sample transport.

Small diameter portion 9 includes funnel shaped transition 8, and boundary 7 separates stem 6 from small diameter bulb portion 9. Small diameter portion 9 is used as a part of the storage area when volumetric pipette 1 is used to transport the sample. Funnel shaped transition 8 is designed to give small diameter bulb portion 9 a smooth transition without any corners to stem 6, such that the liquid sample is fully dispensed and no part of the liquid sample is trapped inside small diameter portion 9.

Small diameter portion 9 is connected to stem 6 at boundary 7. Boundary 7 can be used by the operator to determine when the desired sample volume has been collected. A sample volume corresponding to the inner volume of stem 6 is collected when the sample level reaches boundary 7. This volume can be easily and accurately determined by visually monitoring boundary 7.

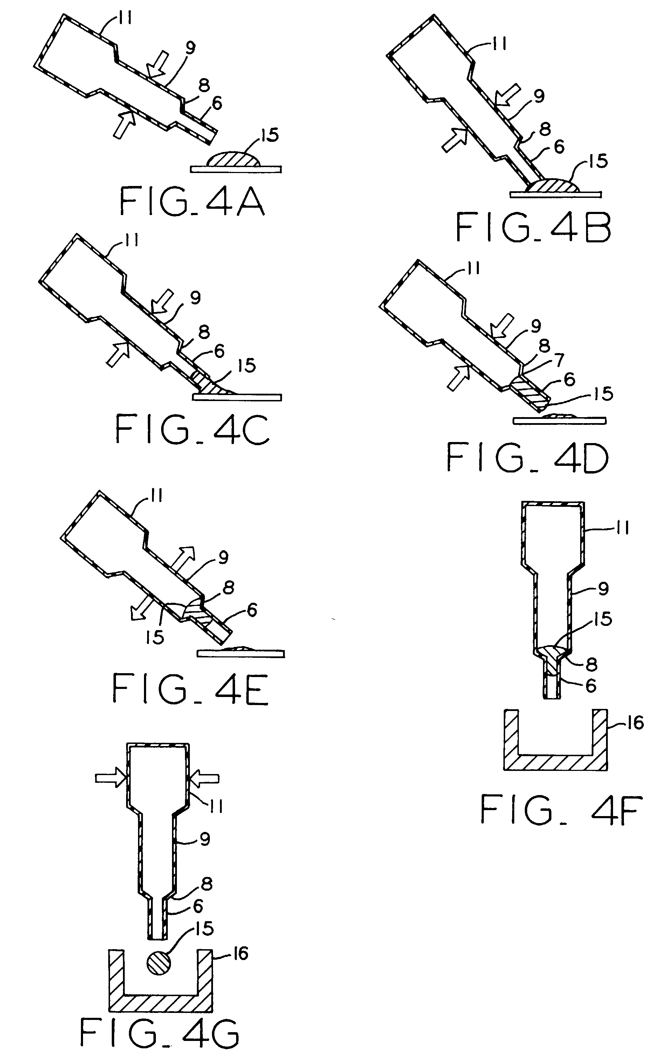

Figs. 4A - 4G show the sequence of steps for drawing, transporting, and dispensing a known sample volume using the inventional pipette.

As shown in Fig. 4A, pipette 1 is placed near the vicinity of sample 15 and small diameter bulb portion 9 is squeezed to displace and expel a volume of air inside volumetric pipette 1. Next, open tip 5 of pipette 1 is placed in contact with the surface of sample 15 while pressure is maintained on small diameter bulb portion 9, as shown in Fig. 4B. Pressure on small diameter bulb portion 9 is then slowly released to draw sample 15 into stem 6 through open tip 5, as shown in Fig. 4C. While sample 15 is being drawn, the operator visually monitors the level of sample 15 in stem 6 and ceases drawing the sample when the level of the sample reaches boundary 7.

After the desired sample volume has been collected, open tip 5 of pipette 1 is removed from the sample while constant pressure is maintained on small diameter bulb portion 9, as shown in Fig. 4D. Next, pressure on small diameter bulb portion 9 is further released to draw sample 15 further into pipette 1, as shown in Fig. 4E. As a result, one end of sample 15 is drawn into funnel shaped portion 8 of small diameter bulb portion 9 and the other end of sample 15 is drawn away from open tip 5. The movement of sample 15 further into pipette 1 ensures that no part of sample 15 is lost while transporting sample 15 to the test location. While sample 15 is being transported, the operator is able to fully release the grip on small diameter bulb portion 9 and maintain a grip on pipette 1 using tab 12.

Fig. 4F shows pipette 1 placed over test device 16 after being transported to a test location. Test device 16 represents any collection device used in connection with a number of diagnostic tests. These diagnostic tests include but are not limited to Hemoglobin, Hematocrit, Glucose, BUN, Cholesterol and Triglyceride.

Finally, sample 15 is dispensed from pipette 1 onto test device 16 by squeezing large diameter bulb portion 11, as shown in Fig. 4G. The volume of air displaced by the squeezing of large diameter portion 11 is sufficient to ensure that all of the sample in pipette 1 is dispensed onto test device 16. Funnel shaped portion 8 ensures that the entire sample is dispensed and that no part of the sample is trapped within small diameter portion 9.

The exemplary embodiment of this invention comprises a pipette having the following dimensions:
a stem volume of 10 microliters, outside diameter of 1.25 millimeters, and length of 12.5 millimeters;
a funnel shaped transition length of 5 millimeters;
a narrow diameter bulb diameter of 3.3 millimeters, and length of 10 millimeters;
a wide diameter bulb diameter of 8 millimeters, and length of 10 millimeters.

While this invention has been described as having a preferred design, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A unitary pipette (1) for collecting, transporting and dispensing a desired measured sample volume comprising: a generally cylindrical stem (6); and a bulb portion (9, 11) connected to said stem, characterized in that said stem has a length and inner diameter to provide a desired sample volume, said bulb portion includes a first bulb portion (9) connected to a second bulb portion (11), said first bulb portion has a larger inner diameter than said stem inner diameter, said second bulb portion has a larger inner diameter than said first bulb portion, and said second bulb portion is sealed at the end opposite the connection to said first bulb portion.

2. The pipette of claim 1 characterized in that said pipette is made of a material which is flexible and durable, said pipette material being easily moldable to provide a low cost device with the features needed for ease of collection, transport and dispense.

3. The pipette of claim 1 characterized in that said pipette is made of polyethylene.

4. The pipette of claim 1 characterized by a frustoconical transition portion (8) between said stem and said first bulb portion.

5. The pipette of claim 1 characterized by a tab portion (12) disposed on said second bulb portion.

6. The pipette of claim 1 characterized in that said desired measured sample volume is in the range of about 2 to 30 microliters.

7. A method for drawing, transporting and dispensing a desired sample volume of a liquid, comprising the steps of: providing a pipette (1) with a bulb portion (9, 11); applying inward pressure to the bulb portion to expel air inside the pipette; placing an open tip (5) of the pipette against a liquid sample (15); releasing the inward pressure on the bulb portion until the desired liquid sample volume is drawn into the pipette; removing the open tip of the pipette from the liquid sample; further releasing the inward pressure on the bulb portion to draw the liquid sample further into the pipette; transporting the pipette to a desired location (16) and placing the tip of the pipette over the desired location; and applying inward pressure on the bulb portion until the liquid sample is fully dispensed from the pipette, characterized by: providing a unitary pipette having a stem (6) with a length and inner diameter determined according to the desired sample volume, a first bulb portion (9) connected to the stem and a second, wide diameter bulb portion (11) connected to the first bulb portion, the second bulb portion sealed at the end opposite the connection to the first bulb portion; applying inward pressure to the first bulb portion to expel air inside the pipette; placing an open tip of the stem against a liquid sample; releasing the inward pressure on the bulb portion until the liquid sample volume completely fills the stem; removing the open tip of the stem from the liquid sample and further releasing the inward pressure on the first bulb portion to draw the liquid sample further into the pipette; transporting the pipette; and applying inward pressure on the second bulb portion until the liquid sample is fully dispensed from the pipette.

8. The method of claim 7 characterized by, prior to placing an open tip of the stem against a liquid sample, the step of puncturing the skin of a finger to draw capillary blood to the surface of the finger and/or puncturing the skin of a heel to draw capillary blood to the surface of the heel to create a liquid sample volume.

9. The method of claim 7 characterized by drawing a sample volume in the range of 2 to 30 microliters to perform a diagnostic test.

10. The method of claim 7 characterized by providing a pipette having a tab portion (12) disposed on the second bulb portion and transporting the pipette to a desired location by gripping the tab portion and releasing the first bulb portion.
